# EUROPÄISCHE PATENTSCHRIFT

(11) **EP 1 888 149 B1**
(45) Veröffentlichungstag und Bekanntmachung des Hinweises auf die Patenterteilung: **25.02.2009**
(21) Anmeldenummer: 06721948.5
(22) Anmeldetag: 05.05.2006
(51) Int. Cl.: A61M 5/315, A61M 5/24

(54) **GEWINDESTANGE UND DOSIERVORRICHTUNG FÜR EINE INJEKTIONSVORRICHTUNG**
THREADED ROD AND DOSING DEVICE FOR AN INJECTION DEVICE
TIGE FILETEE ET DISPOSITIF DE DOSAGE POUR UN DISPOSITIF D'INJECTION

(30) Priorität: 24.05.2005 DE 102005023821
(43) Veröffentlichungstag der Anmeldung: 20.02.2008
(73) Patentinhaber: TecPharma Licensing AG, 3401 Burgdorf (CH)
(72) Erfinder: MOSER, Ulrich, CH-3412 Heimiswil (CH); BURREN, Stefan, CH-3047 Bremgarten (CH); SCHRUL, Christian, CH-3400 Burgdorf (CH)
(74) Vertreter: Schwabe - Sandmair - Marx
(86) Internationale Anmeldenummer: PCT/CH2006/000246
(87) Internationale Veröffentlichungsnummer: WO 2006/125329

(56) Entgegenhaltungen:
- WO-A-94/07562
- FR-A- 2 612 782
- US-A1- 2002 123 717
- US-A1- 2003 089 743

## Beschreibung

Die vorliegende Erfindung bezieht sich auf eine Dosiervorrichtung für eine Injektionsvorrichtung, insbesondere auf eine Dosiervorrichtung, mit welcher eine Dosis oder Menge einer aus- oder mit Hilfe der Injektionsvorrichtung abzugebenden Substanz eingestellt werden kann.

Aus der DE 202 09 051 U1 ist ein Injektionsgerät mit endpositionsblockiertem Dosiseinstellglied bekannt, wobei das Injektionsgerät ein Dosiseinstellglied aufweist, das für die Auswahl der Produktdosis relativ zu dem Gehäuse eine rotatorische Bewegung in eine erste Drehrichtung bis in eine Endposition und in eine entgegengesetzte Drehrichtung ausführen kann und das mit einer Fördereinrichtung, mit welcher eine ausgewählte Produktdosis aus einem Reservoir ausschüttbar ist, so gekoppelt ist, dass die Fördereinrichtung bei einer Betätigung die mittels des Dosiseinstellgliedes ausgewählte Produktedosis ausschüttet. Eine Verdrehsicherungseinrichtung verhindert eine rotatorische Bewegung des Dosiseinstellgliedes in die erste Drehrichtung über eine Endposition hinaus.

Die EP 0 828 527 B1 offenbart ein Injektionsgerät mit einer längsverschiebbaren Vorschubhülse, wobei ein Dosisaufdruck zum Ablesen einer zu injizierenden Dosis vorgesehen ist und ein Mechanismus vorgesehen ist, der ein Laden des Injektionsgeräts durch Herausziehen einer Schubstange dann verhindert, wenn der Vorrat einer Ampulle vollständig aufgebraucht ist, wobei die Dosiervorrichtung eine tatsächlich mögliche verabreichbare Dosis an einer im Bereich eines oberen Endes der Vorschubhülse angebrachten Dosisskala ablesbar ist.

Aus der deutschen Patentanmeldung Nr. 10 2005 001 159.4 ist eine Dosiervorrichtung für eine Injektionsvorrichtung mit Übersetzung bekannt, mit welcher insbesondere eine kleine Dosismenge exakt eingestellt und ausgeschüttet werden kann.

Die WO 2004/078239 A1 offenbart eine Medikamentenverabreichungsvorrichtung mit einem Gehäuse mit Innengewinde, einer Dosiswählhülse mit einem Gewinde, welches in das Innengewinde des Gehäuses eingreift, einer Drehhülse, welche lösbar mit der Dosiswählhülse verbunden ist, und einer Kupplung, welche zwischen der Dosiswählhülse und der Drehhülse angeordnet ist, wobei sich beide Hülsen in Bezug auf das Gehäuse drehen können, wenn die Dosiswählhülse und die Drehhülse gekoppelt sind. Wenn die Dosiswählhülse und die Drehhülse entkoppelt sind, wird eine Rotation der Dosiswählhülse in Bezug auf das Gehäuse ermöglicht, während eine Drehung der Drehhülse in Bezug auf das Gehäuse gesperrt ist, wodurch eine axiale Bewegung der Drehhülse ermöglicht wird, so dass eine Kraft in die longitudinale Richtung zu dem proximalen Ende der Medikamentenverabreichungsvorrichtung übertragen wird.

Aus der WO 94/07562 ist eine Gewindestange mit in Umfangsrichtung angebrachten Rastelementen bekannt.

Die US 2002/0123717 A1 und FR 2 612 782 offenbaren, dass in einer Gewindestange Vertiefungen vorgesehen sein können.

Aus der US 2003/089743 A1 ist bekannt, dass Gewindeabschnitte einen Gewindedurchmesser D1 haben, weitere Gewindeabschnitte einen Durchmesser D2 definieren und weitere Gewindeabschnitte einen variablen Durchmesser stetig ansteigend von D3 bis D4 haben.

Es ist eine Aufgabe der vorliegenden Erfindung, eine Gewindestange und Dosiervorrichtung zur Einstellung und/oder Abgabe einer aus einer Injektionsvorrichtung abzugebenden Dosis, sowie eine Injektionsvorrichtung mit einer solchen Dosiervorrichtung vorzuschlagen, mit welcher eine sichere Einstellung der Dosis ermöglicht wird und das Risiko einer Fehlbedienung bei der Einstellung und/oder Abgabe der Dosis verringert wird.

Diese Aufgabe wird durch die Gegenstände der unabhängigen Ansprüche gelöst. Vorteilhafte Ausführungsformen ergeben sich aus den Gegenständen der abhängigen Ansprüche.

Die Erfindung bezicht sich auf eine Gewindestange, die einen Druck auf einen Stopfen in einem Reservoir einer Injektionsvorrichtung ausüben kann, welches eine abzugebende Substanz enthält und auf eine Dosiervorrichtung zur Einstellung einer aus einer Injektionsvorrichtung abzugebenden Dosis oder Substanz mit einer solchen Gewindestange.

Die Injektionsvorrichtung weist eine Drehhülse auf, mit welcher eine aus der Injektionsvorrichtung abzugebende Dosis durch Drehung der Drehhülse, also z.B. durch ein Herausschrauben oder Einschrauben der Drehhülse in die Injektionsvorrichtung, aufgezogen oder eingestellt werden kann. Die Gewindestange trägt an ihrer Aussenseite ein Gewinde, welches als umlaufendes Gewinde ausgebildet sein kann oder aus mehreren z.B. axial und/oder in Umfangsrichtung versetzten Gewindeteilstücken bestehen kann, und ist bevorzugt in der Injektionsvorrichtung und/oder der Einstellhülse geführt und in diesen axial bewegbar, z.B. durch ein auf der Innenseite der Injektionsvorrichtung oder Einstellhülse vorgesehenes Drehbegrenzungselement. Das Gewinde der Gewindestange kann von dieser vorstehen z.B. als Steg oder vertieft z.B. als Nut ausgebildet sein.

An der Injektionsvorrichtung und/oder der Einstell- und/oder der Drehhülse ist ein z.B. mit dem Gehäuse der Injektionsvorrichtung fest verbundenes Drehbegrenzungselement vorgesehen, welches als Drehraster, Verdrehsicherung oder Rückdrehsicherung ausgebildet sein kann. Ein Drehbegrenzungselement im Sinne der Erfindung ist ein Element, welches im Zusammenwirken mit einem durch oder in dem Drehbegrenzungselement geführten Element, wie z.B. der Gewindestange, eine Drehung des geführten Elements, wie z.B. der Gewindestange, nur in eine Richtung ermöglicht und in die entgegengesetzte Richtung sperrt oder verhindert. Hierzu können auf der Gewindestange entsprechende Gegenelemente vorgesehen sein. Beispielsweise kann mit einer Kombination von Rastnasen oder -nocken auf dem Drehbegrenzungselement oder der Gewindestange, welche in entsprechende Einraststellen, wie z.B. eine Rasterung oder Zahnung, der Gewindestange oder des Drehbegrenzungselementes eingreifen, eine Drehbegrenzung realisiert werden, welche z.B. nach dem Prinzip einer bekannten Ratsche funktioniert. Eine Rastnase kann beispielsweise auf einem z.B. in radiale Richtung vorgespannten elastischen Element, wie z.B. einem Rastarm, der in Umfangsrichtung des geführten Elementes auf diesem oder dem Drehbegrenzungselement angeordnet ist, angeordnet sein oder direkt auf dem jeweiligen Element ohne elastisches Element angeordnet sein und z.B. eine Abschrägung auf einer Seite aufweisen, um ein Herausgleiten aus einer Einraststelle zu ermöglichen und eine der abgeschrägten Seite gegenüberliegenden nicht abgeschrägten Seite haben, welche eine Drehung in der zum Herausgleiten des Rastelements erforderlichen entgegengesetzten Richtung verhindert.

Das auf der Gewindestange vorgesehene Gewinde weist eine Zahnung auf dem Gewindegang oder Gewindesegment auf, in welche ein oder mehrere Rastelemente eines oder mehrerer Drehbegrenzungselemente eingreifen können, um so eine Drehung der Gewindestange relativ zum Drehbegrenzungselement nur in eine Richtung zu ermöglichen. Eine Gewindestange kann also z.B. in eine mit einem Drehbegrenzungselement verbundene Einstellhülse nur eingeschraubt oder nur herausgedreht werden, wobei die jeweilige Gegendrehbewegung durch das Drehbegrenzungselement blockiert wird.

Die Injektionsvorrichtung weist bevorzugt ebenso wie die Drehhülse ein Drehbegrenzungselement auf, welches eine Drehung einer Gewindestange in die gleiche Richtung ermöglicht und in die entgegengesetzte Richtung blockiert. Da die Drehhülse drehbar und bevorzugt durch ein Gewinde geführt in der Injektionsvorrichtung gelagert ist und aus dieser zum Aufziehen der Injektionsvorrichtung herausgezogen oder herausgeschraubt und zum Abgeben einer Dosis wieder eingeschoben oder eingeschraubt werden kann, ermöglicht es eine an der Drehhülse vorgesehene Rückdrehsicherung, dass während des Herausdrehens der Drehhülse aus der Injektionsvorrichtung die Gewindestange von der Drehsicherung der Injektionsvorrichtung gehalten wird, während die Drehsicherung der Drehhülse eine Herausdrehbewegung aus der Injektionsvorrichtung ermöglicht. Wird die Drehhülse wieder in die Injektionsvorrichtung eingeschraubt, so greift das Drehbegrenzungselement der Drehhülse in die Gewindestange ein und bewirkt, dass die Drehhülse die Einschraubbewegung in die Injektionsvorrichtung auf die Gewindestange überträgt und diese mitnimmt, wobei ein Mitdrehen der Gewindestange durch das Drehbegrenzungselement der Injektionsvorrichtung ermöglicht wird, welches eine Drehung der Gewindestange während des Herausschraubens der Drehhülse aus der Injektionsvorrichtung blockiert hat.

Durch ein Mitdrehen der Gewindestange während des Eindrehens der Drehhülse kann die zum Beispiel in einem Gewinde der Injektionsvorrichtung geführte Gewindestange in der Injektionsvorrichtung distal verschoben werden, um z.B. einen Stopfen eine definierte Strecke in ein Behältnis, wie z.B. eine Ampulle, einzuschieben und so ein in dem Behältnis enthaltenes Fluid oder eine Substanz aus diesem zu verdrängen und so durch eine Abgabeöffnung des Behältnisses z.B. an einen Patienten abzugeben.

An der Drehhülse kann ein Drehknopf angebracht sein, welcher relativ zur Drehhülse gedreht werden kann, so dass ein Benutzer z.B. die Drehhülse mittels eines Zuges an dem Drehknopf aus der Injektionsvorrichtung herausziehen oder herausdrehen kann, ohne dass sich der Drehknopf relativ zur Injektionsvorrichtung dreht, wobei die Drehhülse drehbar in dem Drehknopf gehalten wird. Ebenso ist ein Einschieben oder Eindrehen bzw. Einschrauben der Drehhülse in die Injektionsvorrichtung durch einen Druck auf den Drehknopf möglich, ohne dass sich der Drehknopf relativ zur Injektionsvorrichtung bewegt.

Die Erfindung wird nachfolgend anhand bevorzugter Ausführungsbeispiele beschrieben. Es zeigen:
- Fig. 1A: eine Seitenansicht einer Injektionsvorrichtung;
- Fig. 1B: eine Schnittansicht entlang der Linie B-B in Fig. 1A;
- Fig. 1C: eine Draufsicht auf die in Fig. 1A gezeigte Injektionsvorrichtung;
- Fig. 1D: eine Schnittansicht entlang der Linie D-D in Fig. 1C;
- Fig. 2A - 2H: die Stellungen der Dosiervorrichtung beim Einstellen einer Dosis, Aufziehen der Injektionsvorrichtung und der Abgabe einer Dosis;
- Fig. 3A und 3B: eine Ausführungsform der erfindungsgemässen Drehsicherung; und
- Fig. 4A - 4C: eine Ausführungsform einer weiteren Drehsicherung.

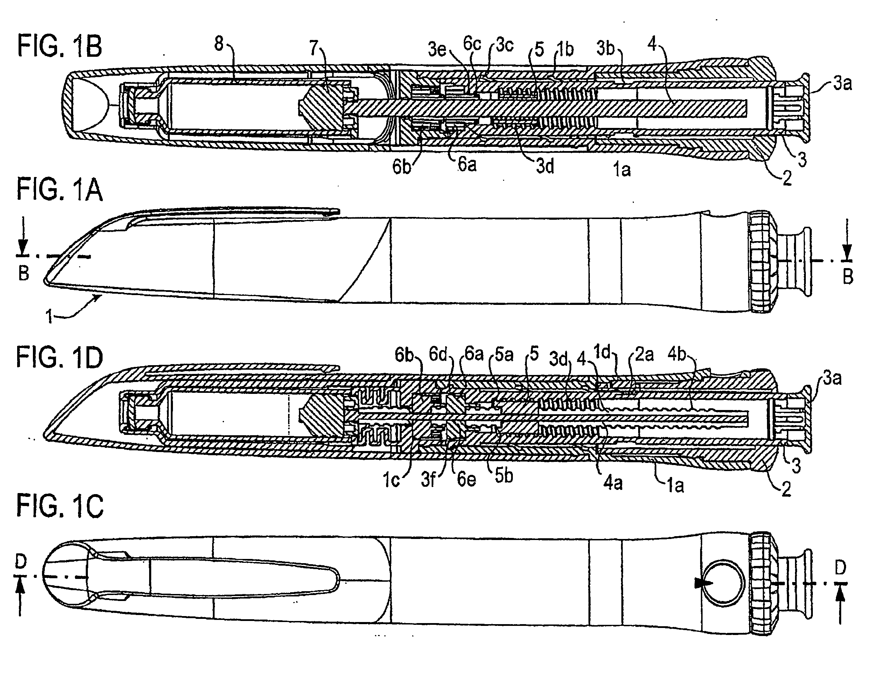

Fig. 1A zeigt eine Seitenansicht einer Injektionsvorrichtung (1), deren Schnitt entlang der Linie B-B in Fig. 1B dargestellt ist.

Die Injektionsvorrichtung (1) weist ein Gehäuse oder Gehäuseelement (1a) auf, in welchem eine Einstellhülse (2) zum einmaligen Vordosieren oder Einstellen der Injektionsvorrichtung z.B. durch einen Arzt oder auch zum mehrmaligen Einstellen verschiedenen Dosismengen drehbar angeordnet ist. Innerhalb der Einstellhülse (2) ist eine Drehhülse (3) drehbar gelagert, welche ein Aussengewinde (3c) hat, das in ein Innengewinde (1b) der Injektionsvorrichtung (1) oder eines Gehäuseteils (1a) der Injektionsvorrichtung eingreift. Die Drehhülse (3) weist ein Innengewinde (3d) auf, in welches ein Aussengewinde (5a) eines Sperrelementes 5 eingreift, so dass die Drehhülse (3) im Gewindeeingriff mit der Injektionsvorrichtung (1) bzw. einem Gehäuse (1a) davon und im Gewindeeingriff mit dem Sperrelement (5) ist. Das Sperrelement (5) ist verdrehgesichert aber axial verschiebbar auf einer Gewindestange (4) gelagert und weist zwei gegenüberliegende Führungselemente (5b) auf, die in eine in axialer Richtung längs der Gewindestange (4) verlaufende Führungsrille (4a) eingreifen, so dass eine Drehung der Gewindestange (4) immer auf das Sperrelement (5) übertragen wird, welches sich mit der Gewindestange (4) mitdreht bzw. welches auch eine Drehung der Gewindestange 4 blockieren kann, wenn es z.B. vollständig in die Drehhülse 3 eingeschraubt ist.

Am distalen Ende der Drehhülse (3) ist ein Drehbegrenzungselement (6a) vorgesehen, welches so mit der Drehhülse (3) verbunden ist, dass sich das Drehbegrenzungselement (6a) in axialer Richtung nicht von der Drehhülse (3) lösen kann, wobei das Drehbegrenzungselement (6a) durch Schnapper oder Halteelemente (6c) so von einem Mitnehmer (3e) der Drehhülse (3) gehalten wird, dass das Drehbegrenzungselement (6a) zwar drehbar relativ zur Drehhülse (3) gelagert ist, jedoch in axialer Richtung nicht relativ zur Drehhülse (3) verschoben werden kann.

Das Drehbegrenzungselement (6a) weist radial nach aussen durch Federarme vorgespannte Rastelemente (6d) auf, die in eine umlaufend an der Innenseite des distalen Endes der Drehhülse (3) vorgesehene Zahnung oder Rasterung (3f) eingreifen, wobei die Zahnung (3f) und die Rastelemente (6d) so ausgebildet sind, dass eine Drehung des Drehbegrenzungselementes (6a) in der Drehhülse (3) nur in einer Richtung möglich ist, in welcher die Rastelemente (6d) aus dem Eingriff mit der Zahnung (3f) herausgleiten können, während eine Drehung in die Gegenbewegung durch eine entsprechende Ausbildung der Rastelemente (6d) verhindert wird, welche in die Zahnung (3f) eingreifen und eine Gegenrichtung blockieren, wobei von dem einseitigen Drehbegrenzungseffekt einer an sich bekannten Ratsche Gebrauch gemacht wird.

Das Drehbegrenzungselement (6a) weist ebenso wie das Sperrelement (5), einander gegenüberliegende Führungselement (6e) auf, welche in die Axialführung oder Rille (4a) der Gewindestange (4) eingreifen und so relativ zur Gewindestange (4) verdrehgesichert sind, wobei jedoch die Gewindestange (4) axial relativ zum Drehbegrenzungselement (6a) verschoben werden kann.

Ein weiteres Drehbegrenzungselement (6b), welches identisch wie das Drehbegrenzungselement (6a) aufgebaut ist, ist drehbar in der Injektionsvorrichtung (1) gelagert und greift mit Rastelementen (6d) in eine auf der Innenseite eines Injektionsvorrichtungsteiles umlaufende Zahnung (1c) ein, so dass das Drehbegrenzungselement (6b) in axialer Richtung nicht in der Injektionsvorrichtung (1) verschoben werden kann und nur eine Drehung in einer Richtung der Injektionsvorrichtung (1) ermöglicht und in der entgegengesetzten Richtung sperrt.

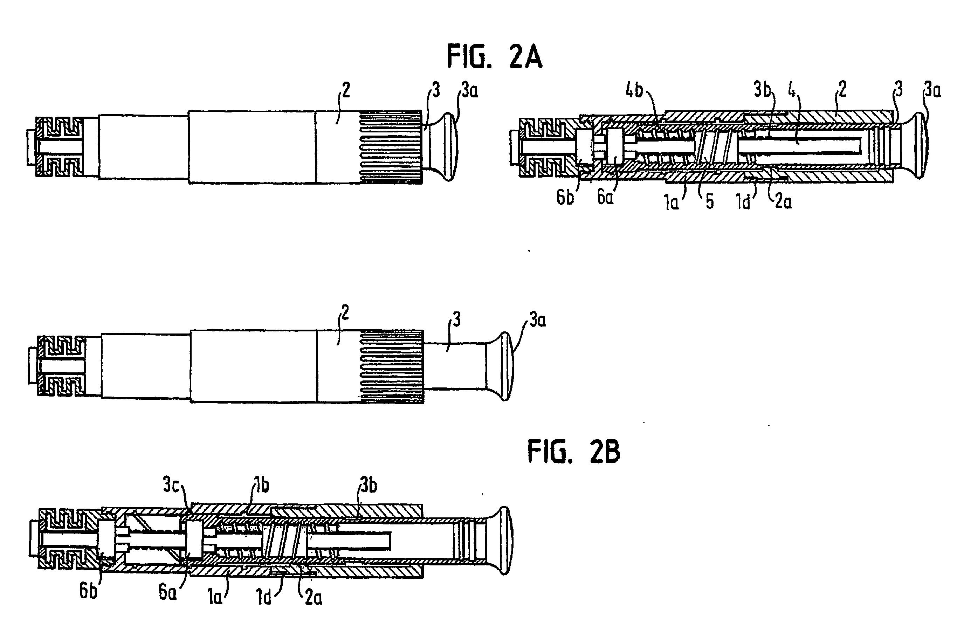

Die prinzipielle Funktionsweise einer Ausführungsform einer Injektionsvorrichtung wird unter Bezugnahme auf die in den Fig. 2A - 2H gezeigten Zustände einer Ausführungsform einer Dosiervorrichtung erläutert. Fig. 2A zeigt in Draufsicht und Querschnittsansicht eine Dosiervorrichtung mit einer Gewindestange (4), welche in der gezeigten Ausführungsform abgeflacht und im Querschnitt etwa rechteckig ist und Gewindesegmente 4b an zwei gegenüberliegenden Seitenflächen trägt, zwischen welchen Teilstücke der Gewindestange (4) liegen, auf welchen keine Gewindeelemente vorgesehen sind. Eine verdrehgesicherte Lagerung der Gewindestange (4) in dem Sperrelement (5) oder den Drehbegrenzungselementen (6a, 6d) wird dadurch erreicht, dass in diesen Elementen etwa der Querschnittsform der Gewindestange (4) entsprechende Durchgangsöffnungen vorgesehen sind.

Durch Drehen an der Drehhülse (3) wird eine gewünschte aufzuziehende und abzugebende Dosis eingestellt. Der Einstellsperrnocken (2a) der Einstellhülse (2) ist nach innen freigestellt und kann in die Freigaberille (3b) geschoben werden, um die Kopplung der Einstellhülse (2) mit dem Gehäuse (1a) durch einen Eingriff des Einstellsperrnockens (2a) in auf der Innenseite des Gehäuses (1 a) liegende Rasterungen aufzuheben.

Nach dem Einstellen der Dosis mittels der frei drehbaren Einstellhülse (2) wird die Injektionsvorrichtung aufgezogen, wie in Fig. 2B in Draufsicht und in Querschnittsansicht gezeigt. Durch Ziehen am frei drehbaren Drehknopf (3a) wird die Injektionsvorrichtung mit der vorher mittels der Einstellhülse (2) eingestellten Dosis von z.B. 2.6 Einheiten aufgezogen oder geladen. Die Drehhülse (3) wird zusammen mit dem frei drehbaren Drehknopf (3a) in proximale Richtung (in Fig. 2B nach rechts) gezogen und dreht sich aufgrund des Gewindeeingriffs des Aussengewindes (3c) der Drehhülse (3) im Innengewinde (1c) des Injektionsvorrichtungsgehäuses (1a).

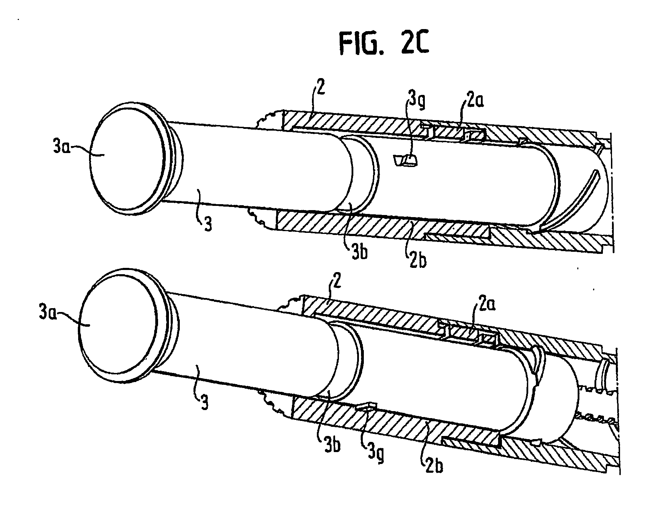

Eine auf der Innenseite der Einstellhülse (2) vorgesehene Anschlagleiste (2b), deren radiale Position durch den Einstell- bzw. Drehvorgang der Einstellhülse (2) festgelegt wurde, begrenzt eine maximal mögliche Dreh- oder Aufziehbewegung der Drehhülse (3), welche auf ihrer Aussenseite ein Anschlagelement (3g) z.B. in Form eines vorstehenden Nockens trägt. Wie in Fig. 2C gezeigt, kann die Drehhülse (3) soweit aus der Injektionsvorrichtung (1) herausgedreht werden, bis das Anschlagelement (3g) an der Anschlagsleiste (2b) der Einstellhülse anliegt und somit ein weiteres Drehen oder Herausziehen der Einstellhülse (2) verhindert.

Wie aus den Fig. 2B und 2C ersichtlich ist, wird während eines Aufziehvorganges beim Herausziehen der Drehhülse (3) die auf der Drehhülse (3) vorgesehene Freigaberille (3b) von dem Einstellsperrnocken (2a) der Einstellhülse (2) wegbewegt, so dass der Einstellsperrnocken (2a) nicht mehr radial nach innen ausweichen kann und somit durch einen Eingriff in die auf der Innenseite des Gehäuses (1a) vorgesehene Rasterung (1d) eingreift und somit eine radiale oder Verdrehbewegung der Einstellhülse (2) relativ zur Injektionsvorrichtung (1) bzw. dem Gehäuseelement (1 a) verhindert. Eine Veränderung der mittels der Einstellhülse (2) einstellbaren Dosis kann somit während eines Aufzieh- und Abgabevorganges bei zumindest zum Teil herausgezogener Drehhülse (3) nicht mehr vorgenommen werden.

Im Inneren der Einstellhülse (2) dreht sich während eines Aufziehvorganges das Sperrelement (5) relativ zur Einstellhülse (2) in dieses hinein, d.h. das Sperrelement wird relativ zur Einstellhülse (2) in distale Richtung der Einstellhülse (2) bewegt, da das Sperrelement verdrehgesichert auf der Gewindestange (4) gelagert ist und die Drehhülse (3) beim Herausdrehen relativ zum Sperrelement (5) gedreht wird, was durch den Gewindeeingriff des Sperrelementes (5) in das Innengewinde (3d) der Drehhülse (3) beim Herausdrehen für eine Relativbewegung des Sperrelementes (5) in axialer Richtung der Drehhülse (3) sorgt.

Während des Aufziehvorganges der Injektionsvorrichtung, d.h. während des Herausdrehens der Drehhülse (3), verhindert das vordere mit der Injektionsvorrichtung verbundene Drehbegrenzungselement (6b), dass die Gewindestange (4) beim Aufziehen der Injektionsvorrichtung mitgedreht wird, während das mit der Drehhülse (3) verbundene Drehbegrenzungselement (6a) eine Drehbewegung der Drehhülse (3) relativ zur durch das Drehbegrenzungselement (6b) gehaltenen Gewindestange (4) ermöglicht.

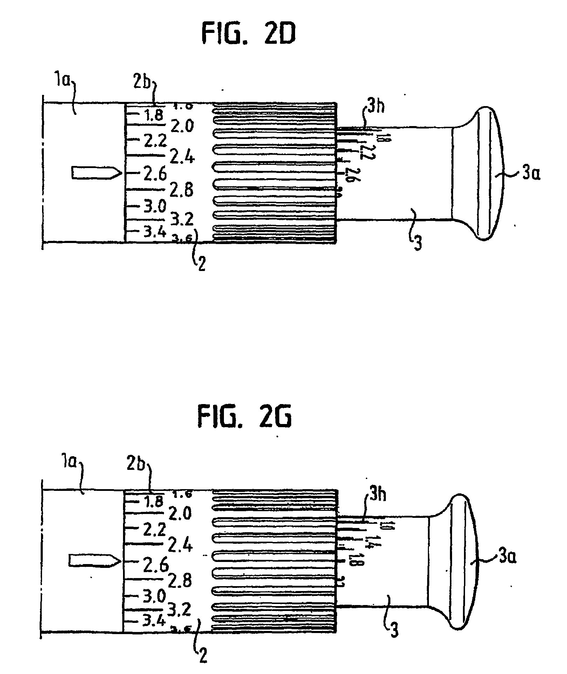

Wie in Fig. 2D gezeigt, ist auf der Aussenseite der Einstellhülse (2) in Umfangsrichtung eine Markierung vorgesehen, wobei in der in Fig. 2D gezeigten Einstellung eine Dosis von 2.6 eingestellt wurde, indem die Einstellhülse (2) so gedreht wird, dass eine der Dosis 2.6 durch einen Strich markierte zugeordnete Drehposition an einem durch einen Pfeil dargestellten Markierungselement auf dem Gehäuse (1a) liegt. Im vollständig herausgezogenen Zustand der Drehhülse (3), welche durch den Anschlag des Anschlagelementes (3g) an der Anschlagleiste (2b) bestimmt wird, ist die Drehhülse (3) so weit aus der Injektionsvorrichtung herausgedreht, dass eine auf der Umfangsseite der Drehhülse (3) vorgesehene Markierung der tatsächlich aufgezogenen Dosis 2.6 vollständig zu erkennen ist, wobei die Markierung 2.6 auf der Drehhülse (3) in axialer Verlängerung zur Markierung 2.6 auf der Einstellhülse (2) liegt, um einem Benutzer so anzuzeigen, dass die eingestellte Dosis auch tatsächlich aufgezogen wurde. Ein Benutzer kann einen Vergleich der eingestellten mit der aufgezogenen Dosis durch einen Blick auf die Injektionsvorrichtung vornehmen, ohne eine z.B. auf einer Seite der Injektionsvorrichtung angezeigte eingestellte Dosis mit einer auf einer anderen Seite der Injektionsvorrichtung angezeigte "tatsächlich aufgezogene" Dosis vornehmen zu müssen.

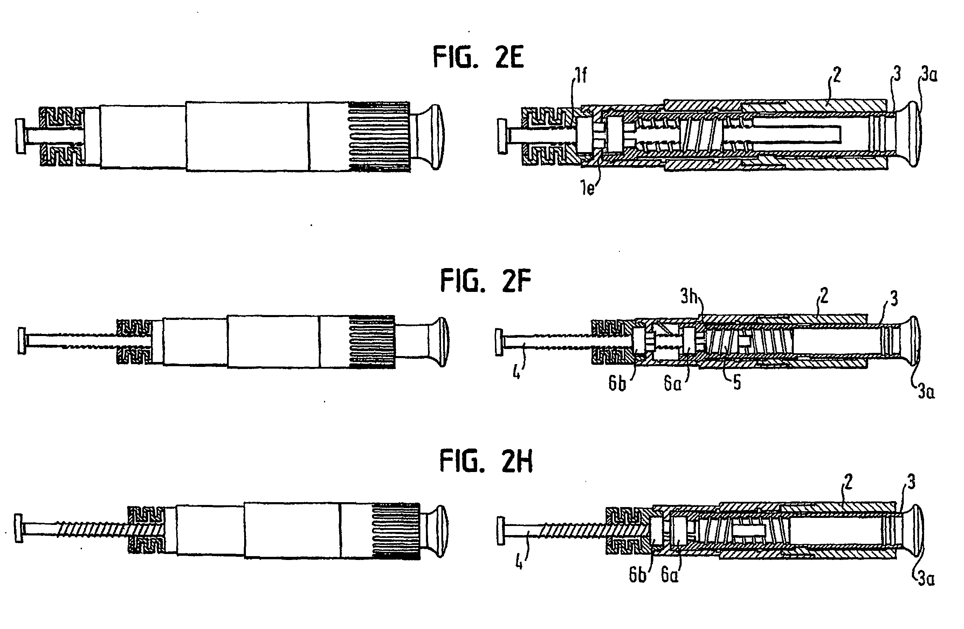

Zum Ausschütten einer voreingestellten Dosis kann der Drehknopf (3a) gedrückt werden, wodurch die drehbar in dem Drehknopf (3a) gelagerte Drehhülse (3) in die Injektionsvorrichtung eingedreht wird, wie in Fig. 2E gezeigt, bis ein vorderes oder distales Ende der Einstellhülse (2) an einem Innenanschlag (1 e) der Injektionsvorrichtung (1) anliegt. Beim Eindrehen der Einstellhülse (2) gibt das mit der Injektionsvorrichtung (1) verbundene vordere Drehbegrenzungselement (6b) die Drehbewegung der Gewindestange (4) frei, welche von dem mit der Drehhülse (3) verbundenen Drehbegrenzungselement (6a) gehalten und zusammen mit der Drehhülse (3) relativ zur Injektionsvorrichtung (1) gedreht wird und sich so durch einen Gewindeeingriff in ein Innengewinde (1f) der Injektionsvorrichtung (1) relativ zur Injektionsvorrichtung (1) in distale Richtung schraubt, um einen z.B. an der Vorderseite der Gewindestange (4) anliegenden Stopfen (7), wie in Fig. 1B gezeigt, in eine Ampulle (8) einzuschieben und eine in der Ampulle (8) enthaltene Substanz zu verdrängen und so die voreingestellte Dosis abzugeben.

Nach erfolgtem Abgabevorgang kann die mittels der Drehhülse (3) eingestellte Dosis einfach wieder durch Herausziehen der Drehhülse (3) aufgezogen und abgegeben werden, wie oben beschrieben, ohne dass eine erneute Dosiseinstellung vorgenommen werden muss, da die Einstell- oder Drehposition der Einstellhülse (2) während des Aufzieh- und Abgabevorganges nicht verändert wurde.

Wird das Sperrelement (5) so in die Drehhülse (3) eingesetzt, dass die insgesamt mögliche von dem Sperrelement (5) bis zu einem vorderen Anschlag innerhalb der Drehhülse (3) zurückzulegende Strecke einer gewünschten maximal abzugebenden Substanzmenge entspricht, so kann, falls eine mittels der Einstellhülse (2) eingestellte Dosis grösser als die vorgegebene maximal zulässige Gesamtabgabemenge ist, eine Begrenzung des Aufziehvorganges der Injektionsvorrichtung durch ein Blockieren des Herausdrehens der Drehhülse (3) realisiert werden.

Wird z.B. eine letzte zulässige Dosis aufgezogen, wie in Fig. 2F gezeigt, so wird wiederum die Drehhülse (3) mittels des Drehknopfes (3a) aus der Injektionsvorrichtung (1) herausgezogen bzw. herausgedreht. Gleichzeitig dreht sich im Inneren der Drehhülse (3) das Sperrelement (5) so weit in die Drehhülse (3) hinein, bis das Sperrelement (5) an einem vorderen Anschlag (3h) der Drehhülse (3) liegt und so ein weiteres Herausdrehen der Drehhülse (3) blockiert, die durch den Gewindeeingriff mit dem Aussengewinde (5a) des Sperrelementes (5) gehalten wird, welches sich relativ zur Injektionsvorrichtung nicht drehen kann, da es verdrehsicher auf der Gewindestange (4) gelagert ist, die während des Herauszieh- oder Aufziehvorganges drehsicher von dem vorderen mit der Injektionsvorrichtung(1) verbundenen Drehbegrenzungselement (6b) gehalten wird.

Wie in Fig. 2G gezeigt, kann ein Benutzer leicht erkennen, dass in axialer Verlängerung der auf der Einstellhülse (2) vorgesehenen Markierung 2.6 im blockierten herausgedrehten Endzustand der Drehhülse (3) die maximal noch mögliche aufgezogene Dosis 1.8 angezeigt wird, so dass ein Benutzer leicht ablesen kann, dass die gewünschte eingestellte Dosis nicht mehr abgegeben werden kann.

Wie in Fig. 2H gezeigt, kann durch Drücken auf den Drehknopf (3a) die letzte Dosis ausgeschüttet werden. Durch das vollständig in die Drehhülse (3) eingeschraubte Sperrelement (5), welches an dem vorderen Anschlag (3h) der Drehhülse (3) ansteht, wird verhindert, dass die Injektionsvorrichtung nochmals aufgezogen werden kann.

Durch den Unterschied der Gewindesteigungen des Aussengewindes (4b) der Gewindestange (4) und des Aussengewindes (3c) der Drehhülse (3) kann, wenn z.B. die Gewindesteigung des Aussengewindes (3c) der Drehhülse (3) grösser als die des Aussengewindes (4b) der Gewindestange (4) ist, eine Untersetzung des Abgabevorganges realisiert werden. Die Drehhülse (3) wird während des Aufziehens weiter aus der Injektionsvorrichtung (1) herausgezogen, als die Gewindestange (4) axial in distale Richtung beim Einschieben der Drehhülse (3) verschoben wird, da während des Einschiebe- oder Abgabevorganges die Drehhülse (3) mit der Gewindestange (4) drehgekoppelt ist und beide Element somit die gleiche Drehbewegung ausführen. Da das Aussengewinde (3c) der Drehhülse (3) eine grössere Steigung aufweist als das Aussengewinde (4b) der Gewindestange (4), kann mit einer in axialer Richtung grösseren Aufzieh- und Einschubbewegung der Drehhülse (3) eine in axialer Richtung kleinere Vorschubbewegung der Gewindestange (4) bewirkt werden, was zu einer feineren Dosierung der durch eine Vorschubbewegung der Gewindestange (4) aus einer Ampulle (8) abgegebenen Substanz führt. Ebenso kann das Verhältnis der Steigung des Aussengewindes der Drehhülse (3) zu der des Aussengewindes dem Gewindestange 4 so festgelegt werden, dass eine Übersetzung oder auch keine Über- oder Untersetzung stattfindet.

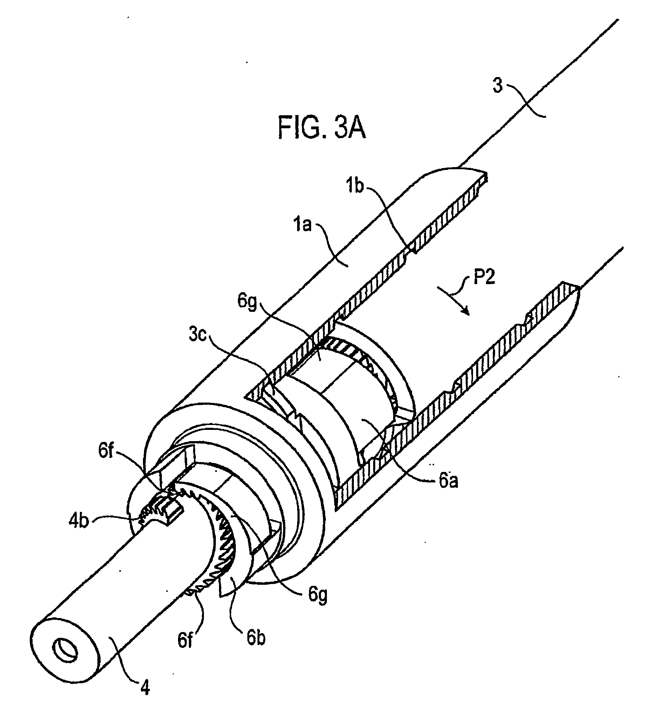

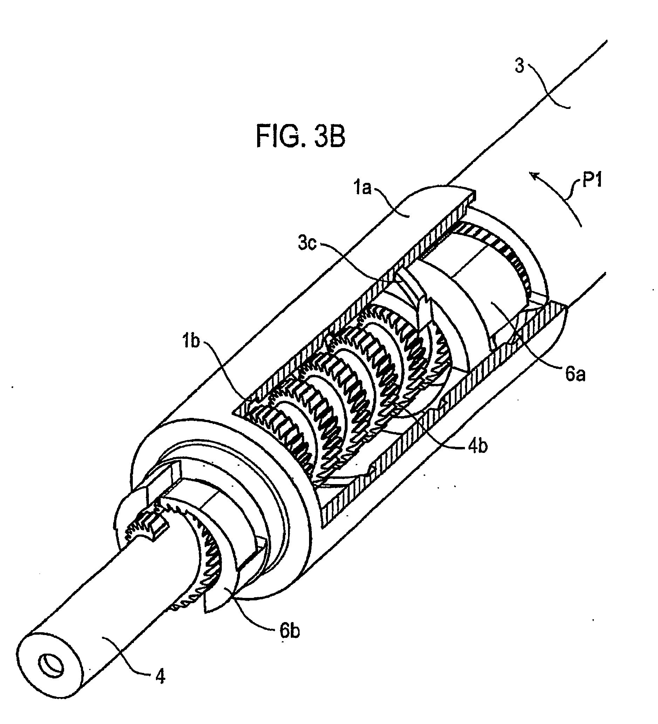

Die Fig. 3A und 3B zeigen eine weitere Ausführungsform der Drehbegrenzungselemente 6a und 6b. In der gezeigten Ausführungsform weist die Gewindestange (4) ein umlaufendes Gewinde (4b) auf, welches in Umfangsrichtung eine Zahnung trägt, bei welcher die einzelnen Zähne so ausgebildet sind, dass an einer Zahnflanke ein Rasten oder Halten mit einer entsprechenden Gegenzahnung oder einem Rastelement möglich ist, während die andere Zahnflanke so ausgebildet ist, dass an einem Rast- oder Gegenelement eine Gleit- oder Verschiebebewegung möglich ist. Beispielsweise kann die ein Eingreifen oder Halten bewirkende Zahnflanke im Wesentlichen in radialer Richtung nach aussen verlaufen, während die eine Dreh- oder Gleitbewegung ermöglichende Zahnflanke relativ zu dieser Zahnflanke schräg oder geneigt ist.

Mit der Injektionsvorrichtung (1) oder einem Gehäuseteil (1a) davon fest verbunden oder darin integriert ist ein Drehbegrenzungselement (6b), welches in der gezeigten Ausführungsform zwei durch jeweils drei Zahnflanken gebildete Rastelemente (6f) aufweist, welche einander gegenüber liegen und an elastischen oder Rastarmen (6g) angebracht sind. Das Gewinde (4b) wird durch ein Innengewinde innerhalb der Injektionsvorrichtung (1) bzw. des Gehäuseteiles (1a) geführt, so dass die Gewindestange (4) durch einen Gewindeeingriff drehbar in der Injektionsvorrichtung (1) bzw. dem Gehäuseteil (1a) gelagert ist und in dieses ein- bzw. ausgeschraubt werden kann. Durch den Eingriff der Rastelemente (6f) in die auf dem Gewinde (4b) der Gewindestange (4) vorgesehene Zahnung ist eine Drehung der Gewindestange (4) relativ zum Gehäuseteil (1a) nur in einer Richtung möglich, wohingegen eine Bewegung in die entgegengesetzte Richtung blockiert wird. In der in Fig. 3A gezeigten Ausführungsform kann die Gewindestange (4) im Uhrzeigersinn gedreht werden, wodurch die Gewindestange (4) in distaler Richtung aus dem Gehäuseteil (1 a) nur herausgeschraubt aber nicht in entgegengesetzter Richtung eingeschraubt werden kann.

Ebenso wie das Drehbegrenzungselement (6b) ist das Drehbegrenzungselement (6a) aufgebaut, welches fest mit der Drehhülse (3) verbunden oder in diese integriert ist und ebenfalls Rastelemente (6f) aufweist, die an elastischen um die Gewindestange (4) umlaufenden Armen (6g) angebracht sind und in die Zahnung des Gewindes (4b) der Gewindestange (4) eingreifen können, um, wie oben beschrieben, eine Drehbewegung zwischen Gewindestange (4) und Drehhülse (3) in einer Richtung zu ermöglichen und in der entgegengesetzten Richtung zu blockieren.

Fig. 3A zeigt die vollständig in die Injektionsvorrichtung eingedrehte Drehhülse (3), welche über ein Aussengewinde (3c) in einem Innengewinde (1b) des Injektionsgerätegehäuses (1 a) geführt ist.

Wird die Injektionsvorrichtung (1) durch ein Herausdrehen der Drehhülse (3) aufgezogen, so wird die Gewindestange (4) von dem Drehbegrenzungselement (6b) verdrehsichert relativ zum Injektionsgerätegehäuses (1 a) gehalten, während das Drehbegrenzungselement (6a) bei der in der durch den Pfeil P1 angegebenen Drehrichtung, welche ein Herausdrehen der Drehhülse (3) aus dem Gehäuse (1 a) bewirkt, nicht in Blockiereingriff mit der Zahnung auf dem Gewindegang (4b) der Gewindestange (4) ist.

Beim Wiedereindrehen der Drehhülse (3) in der in Fig. 3A durch den Pfeil P2 gezeigten Richtung sind die Eingriffselemente des Drehbegrenzungselementes (6a) der Drehhülse (3) im Eingriff mit der Zahnung des Gewindes (4a) der Gewindestange 4, wodurch während des Eindrehens der Drehhülse (3) die Gewindestange (4) drehsicher mit der Drehhülse (3) gekoppelt ist und von dieser mitgenommen und mitgedreht wird. Das Drehbegrenzungselement (6b) der Injektionsvorrichtung (1) ermöglicht eine Drehung der Gewindestange (4) relativ zur Injektionsvorrichtung (1) bzw. dem Gehäuse (1a), so dass die Gewindestange (4) durch die Drehhülse (3) gedreht und in dem Innengewinde der Injektionsvorrichtung (1) bzw. dem Gehäuse (1 a) geführt wird und sich so aus dem Gehäuse (1a) in distale Richtung herausdreht, um eine eingestellte und aufgezogene Dosis abzugeben. Anschliessend kann die Injektionsvorrichtung durch ein Herausdrehen der Drehhülse (3) in der durch den Pfeil P1 in Fig. 3B angegebenen Richtung wieder aufgezogen werden.

Die in den Fig. 3A und 3B gezeigte Ausführungsform der Drehbegrenzungselemente 6a und 6b kann anstatt der unter Bezugnahme auf die Fig. 1 und 2 beschriebenen Drehbegrenzungselemente 6a und 6b in einer Dosiervorrichtung oder Injektionsvorrichtung verwendet werden, wobei alle weiteren Elemente der Injektionsvorrichtung unverändert bleiben können. Z.B. kann das Sperrelement (5) verdrehsicher auf der Gewindestange (4) geführt werden, indem ein oder mehrere Eingriffselemente in axialer Richtung auf der Innenseite des Sperrelementes (5) vorgesehen sind, welche in der Zahnung des Aussengewindes (4b) der Gewindestange (4) geführt werden können, um das Sperrelement (5) axial verschieblich aber drehgesichert auf der Gewindestange (4) zu lagern.

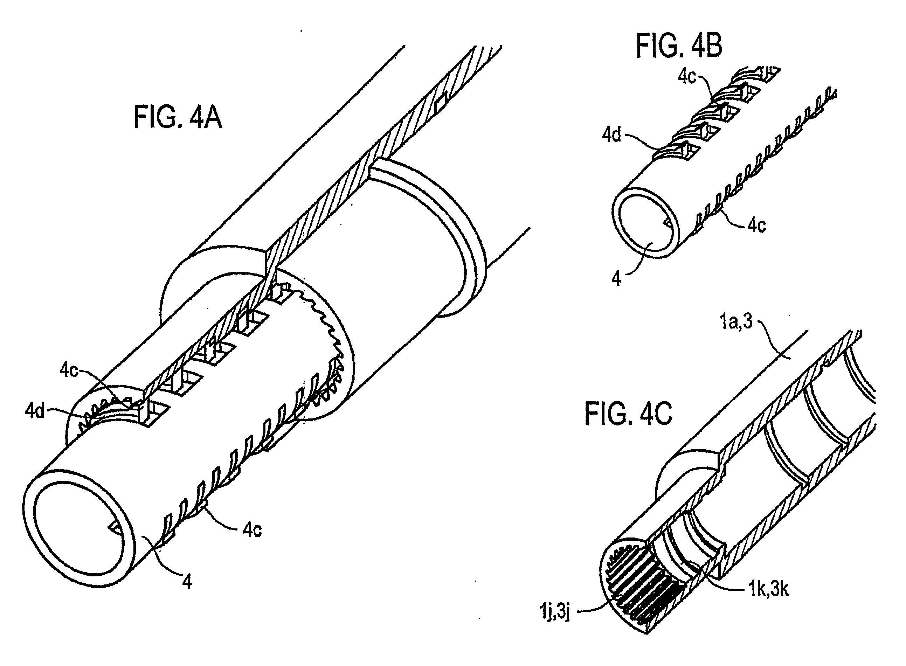

Die Fig. 4A - 4C zeigen eine weitere Ausführungsform eines Drehbegrenzungselementes 6a oder 6b, welches an einem Gehäuse (1 a) der Injektionsvorrichtung oder an der Drehhülse (3) oder in diese Elemente integriert sein kann. Dabei ist der Wirkmechanismus im Vergleich unter Bezugnahme auf die in Fig. 3A und 3B beschriebene Ausführungsform umgekehrt. Auf der Gewindestange (4) sind Gewindeabschnittselemente (4c) vorgesehen, welche durch elastische und z.B. radial nach aussen vorgespannte Tragarme (4d) an der Gewindestange (4) befestigt sind und in eine Innenzahnung (1 j oder 3j) des Injektionsvorrichtungsgehäuses (1a) oder der Drehhülse (3) eingreifen können. Auf der Innenseite des Gehäuses (1a) bzw. der Drehhülse (3) ist axial zu der Rasterung oder Zahnung 1j, 3j versetzt ein Gewinde oder Gewindeabschnitt (1 k, 3k) vorgesehen, in welchem das durch die Rastelemente (4c) und Tragarme 4d-gebildete Aussengewinde der Gewindestange (4) geführt werden kann.

Die Gewindestange (4) kann somit in das Gehäuse (1a) bzw. in die Drehhülse (3) eingedreht werden, wobei die mit der Rasterung (1j, 3j) zusammenwirkenden Rastelemente (4c) der Gewindestange nur eine Drehung der Gewindestange (4) relativ zum Gehäuse (1a) bzw. relativ zur Drehhülse (3) in eine vorgegebene Richtung ermöglichen und in entgegengesetzter Richtung sperren, wie beispielhaft anhand der in den Fig. 3A und 3B gezeigten Ausführungsform erklärt.

Ebenso wie das in den Fig. 3A und 3B gezeigte Drehbegrenzungselement 6a oder 6b kann die in den Fig. 4A - 4C gezeigte Ausführungsform auch in Verbindung mit der in den Fig. 1 und 2 gezeigten Injektionsvorrichtung bzw. Dosiervorrichtung anstelle der dort beschriebenen Drehbegrenzungselemente 6a und 6b verwendet werden.

Das Sperrelement (5) kann verdrehgesichert auf der Gewindestange (4) gelagert werden, indem z.B. auf der Innenseite des Sperrelementes (5) eine Führungsrille oder Nut vorgesehen ist, in welche die Eingriffs- oder Rastelemente (4c) eingreifen können.

## Patentansprüche

1. Gewindestange (4) für eine Injektionsvorrichtung (1) mit einem auf der Außenseite der Gewindestange (4) angeordneten Gewinde (4b, 4c, 4d), wobei das Gewinde (4b, 4c, 4d) zumindest zum Teil eine Zahnung (4b) trägt, wobei einzelne Zähne der Zahnung so ausgebildet sind, dass an einer Zahnflanke ein Rasten oder Halten mit einer entsprechenden Gegenzahnung oder einem Rastelement möglich ist, während die andere Zahnflanke so ausgebildet ist, dass an einem Rast- oder Gegenelement eine Gleit- oder Verschiebebewegung möglich ist, wobei die ein Eingreifen oder Halten bewirkende Zahnflanke im wesentlichen in radialer Richtung nach außen verläuft, und die eine Dreh- oder Gleitbewegung ermöglichende Zahnflanke relativ zu dieser Zahnflanke schräg oder geneigt ist

2. Gewindestange nach Anspruch 1, wobei die Gewindestange eine Abschnittsweise oder vollständig umlaufende Zahnung (4b) trägt.

3. Gewindestange nach Anspruch 2, wobei die Zahnkämme der Zahnung (4b) in axialer Richtung der Gewindestange verlaufen.

4. Gewindestange nach einem der vorhergehenden Ansprüche, wobei das Gewinde als von der Gewindestange (4) vorstehendes Gewinde zum Beispiel als Steg oder in die Gewindestange (4) vertieftes Gewinde zum Beispiel als Nut ausgebildet ist.

5. Dosiervorrichtung für eine Injektionsvorrichtung (1) mit einem Drehbegrenzungselement, welches eine Drehung einer Gewindestange (4) nach einem der vorhergehenden Ansprüche in eine Richtung ermöglicht und in der Gegenrichtung blockiert, mit mindestens einem Rastelement (6f), welches mit einem Gewinde mit Zahnung (4b) der Gewindestange (4) zusammenwirken kann.

6. Dosiervorrichtung nach dem vorhergehenden Anspruch, wobei das Drehbegrenzungselement an einer Drehhülse (3) oder an der Injektionsvorrichtung (1) angebracht ist, in welcher die Gewindestange (4) bevorzugt koaxial geführt oder gelagert werden kann und wobei die Zahnung (1j, 3j) und/oder Eingriffselemente oder Rastelemente so auf der jeweiligen Innenseite bevorzugt in Umfangsrichtung der Gewindestange (4) angeordnet sind, dass diese mit entsprechenden Gegenelementen auf der Gewindestange (4) zusammenwirken können.

7. Injektionsvorrichtung mit mindestens einer Dosiervorrichtung nach einem der vorhergehenden zwei Ansprüche und einer Gewindestange nach einem der Ansprüche 1 bis 4.

## Claims

1. A threaded rod (4) for an injection device (1), comprising a thread (4b, 4c, 4d) arranged on the outer side of the threaded rod (4), wherein the thread (4b, 4c, 4d) at least partially bears a toothing (4b), wherein individual teeth of the toothing are formed such that on one tooth flank, locking or holding with a corresponding counter toothing or with a locking element is possible, while the other tooth flank is formed such that a sliding or shifting movement on a locking element or counter element is possible, wherein the tooth flank which effects the engaging or holding runs substantially outwards in the radial direction, and the tooth flank which enables a rotational or sliding movement is oblique or inclined relative to said tooth flank.

2. The threaded rod according to claim 1, wherein the threaded rod bears a circumferential toothing (4b) in portions or throughout.

3. The threaded rod according to claim 2, wherein the toothed combs of the toothing (4b) run in the axial direction of the threaded rod.

4. The threaded rod according to any one of the preceding claims, wherein the thread is formed as a thread which protrudes from the threaded rod (4), for example as a stay, or is formed as a thread which is receded into the threaded rod (4), for example as a groove.

5. A dosing device for an injection device (1), comprising a rotation limiting element which enables a rotation of a threaded rod (4) according to any one of the preceding claims in one direction and blocks it in the opposite direction and comprises at least one locking element (6f) which can co-operate with a thread comprising the toothing (4b) of the threaded rod (4).

6. The dosing device according to the preceding claim, wherein the rotation limiting element is attached to a rotational sleeve (3) or to the injection device (1), in which the threaded rod (4) can be guided or mounted, preferably coaxially, and wherein the toothing (1j, 3j) and/or the engagement elements or locking elements are arranged on the respective inner side, preferably in the circumferential direction of the threaded rod (4), such that the latter can co-operate with corresponding counter elements on the threaded rod (4).

7. An injection device, comprising at least one dosing device according to any one of the preceding two claims and a threaded rod according to any one of claims 1 to 4.

## Revendications

1. Tige filetée (4) pour un dispositif d'injection (1) avec un filetage (4b, 4c, 4d) disposé sur le côté extérieur de la tige filetée (4), le filetage (4b, 4c, 4d) portant au moins en partie une denture (4b), les différentes dents de la denture étant conçues de telle sorte qu'un enclenchement ou une retenue avec une denture complémentaire correspondante ou un élément d'enclenchement est possible sur un flanc de dent, tandis que l'autre flanc de dent est conçu de telle sorte qu'un mouvement de glissement ou de coulissement est possible sur un élément d'enclenchement ou un élément complémentaire, le flanc de dent provoquant un enclenchement ou une retenue allant essentiellement en direction radiale vers l'extérieur, et le flanc de dent permettant un mouvement de rotation ou de coulissement est en biais ou en incliné par rapport à ce flanc de dent.

2. Tige filetée après la revendication 1, la tige filetée portant une denture (4b) entièrement ou partiellement périphérique.

3. Tige filetée après la revendication 2, les crêtes de dents de la denture (4b) passant dans la direction axiale de la tige filetée.

4. Tige filetée selon l'une des revendications précédentes, le filetage étant conçu comme un filetage faisant saillie à partir de la tige filetée (4), par exemple sous forme de barre, ou comme un filetage en creux dans la tige filetée (4), par exemple comme rainure.

5. Dispositif de dosage pour un dispositif d'injection (1) avec un élément de limitation rotatif qui permet une rotation d'une tige filetée (4) selon l'une des revendications précédentes dans une direction et la bloque dans le sens contraire, avec au moins un élément d'enclenchement (6f) qui peut coopérer avec un filetage avec denture (4b) de la tige filetée (4).

6. Dispositif de dosage selon la revendication précédente, l'élément de limitation rotatif étant fixé sur un manchon rotatif (3) ou sur le dispositif d'injection (1), dans lequel la tige filetée (4) peut être logée ou guidée de préférence de manière coaxiale, et la denture (1j, 3j) et/ou des éléments de mise en prise ou des éléments d'enclenchement étant disposés sur le côté intérieur respectif de préférence dans la direction périphérique, de la tige filetée (4) de sorte qu'ils puissent coopérer avec des éléments complémentaires correspondants sur la tige filetée (4).

7. Dispositif d'injection avec au moins un dispositif de dosage selon l'une des deux revendications précédentes et une tige filetée selon l'une des revendications 1 à 4.
